(19) Europäisches Patentamt
European Patent Office
Office européen des brevets

(11) Publication number: 0 436 851 A1

# (12) EUROPEAN PATENT APPLICATION

(21) Application number: 90123759.4

(22) Date of filing: 11.12.90

(51) Int. Cl.5: C07D 233/54, A61K 31/415, C07D 233/60, C07F 7/18

(30) Priority: 21.12.89 IT 2276789

(43) Date of publication of application: 17.07.91 Bulletin 91/29

(84) Designated Contracting States: AT BE CH DE DK ES FR GB GR IT LI LU NL SE

(71) Applicant: ZAMBON GROUP S.p.A.
Via della Chimica, 9
I-36100 Vicenza(IT)

(72) Inventor: Bertolini, Giorgio
Via A. Costa, 37
I-20099 Sesto San Giovanni (MI)(IT)
Inventor: Casagrande, Cesare
Via Campogallo, 21/67
I-20020 Arese (MI)(IT)
Inventor: Santangelo, Francesco
Via Don Gnocchi, 33
I-20148 Milano(IT)

## (54) Compounds active as inhibitors of the enzyme HMG-CoA reductase and pharmaceutical compositions containing them.

(57) Compounds of formula

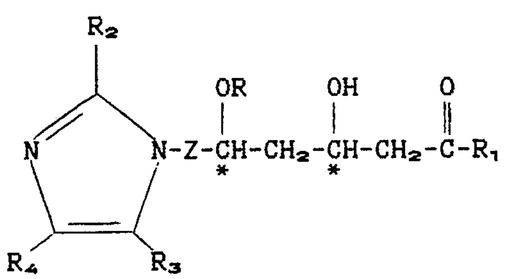

(I)

wherein R, $R_1$, $R_2$, $R_3$, $R_4$ and Z have the meanings reported in the specification, and intermediates for their preparation are described.

The compounds of formula I have antiatherosclerotic activity as inhibitors of the enzyme HMG-CoA reductase and they are useful in the pharmaceutical field.

Compositions for pharmaceutical use containing a compound of formula I as active ingredient are described too.

EP 0 436 851 A1

# COMPOUNDS ACTIVE AS INHIBITORS OF THE ENZYME HMG-COA REDUCTASE AND PHARMACEUTICAL COMPOSITIONS CONTAINING THEM

The present invention relates to compounds with pharmacological activity and, more particularly, it relates to compounds having anti-atherosclerotic activity as inhibitors of 3-hydroxy-3-methyl-glutaryl-CoA reductase (HMG-CoA reductase), the rate controlling enzyme in cholesterol biosynthesis.

Compounds having a complex structure in which a mevalolactone moiety is contained are known to control and inhibit the activity of HMG-CoA reductase. Among these, for example, Lovastatin (Merck Index, XI ed., No. 5460, page 878), isolated as fungal metabolite from Monascus ruber and from Aspergillus terreus, and Mevastatin (Merck Index, XI ed., No. 6088, page 969), isolated as fungal metabolite from Penicillum citrinum may be cited.

Several derivatives of mevalolactone have been studied and, in particular, many arylalkyl derivatives of mevalolactone (wherein the aryl may be also a heterocycle) showed to be useful for treating hyperlipidaemia. Among the disclosed compounds those in which the aryl is an 1-substituted polyhydronaphthalene (European patent application No. 283,123 - Merck & Co. Inc.), a 2-substituted imidazole (International patent application 86/07054 - Sandoz A.G.), a 2-substituted indole (International patent No. application No. 84/02131 - Sandoz A.G.) or an 1-substituted pyrrole (European patent application No. 179,559 - Warner-Lambert Co.) may be cited.

We have now found, and they are an object of the present invention, compounds of formula

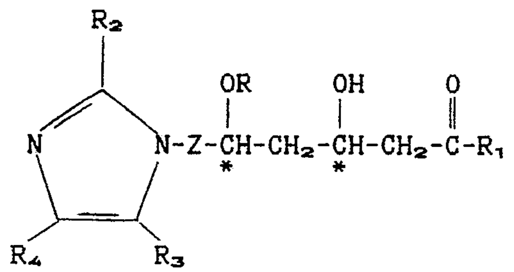

(I)

wherein

R is a hydrogen atom;

$R_1$ is a hydroxyl or an $-OR_5$ group wherein $R_5$ is a $C_1$-$C_4$ alkyl or a benzyl;
or R and $R_1$, together, are a single bond between the oxygen atom and the carbonyl group;

$R_2$, $R_3$ and $R_4$, the same or different, are hydrogen atoms; $C_1$-$C_5$ alkyl or alkoxy groups; $C_2$-$C_5$ alkenyl groups; $C_3$-$C_7$ cycloalkyl or cycloalkylmethyl groups; halogen atoms; $CF_3$ or CN groups; phenyl or benzyl optionally substituted by 1 or 2 substituents selected among halogen atoms and $C_1$-$C_4$ alkyl;a pyridyl or N-oxide thereof;

Z is a $-(CH_2)_m-$ group wherein m is 1, 2 or 3 or a $-CH_2-CH=CH-$group;

the carbon atoms marked by an asterisk are in R or S configuration and in syn relative configuration; and, when $R_1$ is a hydroxyl group, their pharmaceutically acceptable salts.

Specific examples of alkyl, alkoxy, alkenyl and cycloalkyl are methyl, ethyl, propyl, isopropyl, n.butyl, isobutyl, tert-butyl, n.pentyl, isopentyl, methoxy, ethoxy, propoxy, butoxy, isopropoxy, isobutoxy, tert-butoxy, vinyl, allyl, isopropenyl, cyclopropyl, cyclobutyl, cyclopentyl and cyclohexyl.

Examples of suitable pharmaceutically acceptable salts are the salts with an alkali metal, for example sodium or potassium, and with an alkaline earth metal, for example calcium or magnesium, the sodium salt being preferred.

Preferred compounds of formula I are the compounds wherein $R_2$, $R_3$ and $R_4$, the same or different, are hydrogen atoms, $C_1$-$C_4$ alkyl or alkoxy groups, $C_2$-$C_3$ alkenyl groups, $C_3$-$C_5$ cycloalkyl or cycloalkylmethyl groups, phenyl or benzyl optionally substituted by 1 or 2 substituents selected among fluorine, chlorine, bromine atoms and methyl groups.

More preferred compounds of formula I are the compounds wherein $R_2$, $R_3$ and $R_4$, the same or different, are methyl, ethyl, propyl, isopropyl, tert-butyl, methoxy, ethoxy, propoxy, isopropoxy, allyl, isopropenyl, cyclopropyl, cyclobutyl, phenyl, 4-fluorophenyl, 4-chlorophenyl, 4-bromophenyl or 4-methylphenyl; Z is a $-(CH)_2)_m-$ group wherein m is 2 or a $-CH_2-CH=CH-$ group.

Specific examples of preferred compounds according to the present invention are:

6-[2-[4,5-di-(4-fluorophenyl)-2-isopropyl-imidazol-1-yl]-ethyl]-4-hydroxy-tetrahydropyran-2-one
7-[4,5-di-(4-fluorophenyl)-2-isopropyl-imidazol-1-yl]-3,5-dihydroxy-heptanoic acid
6-[3-[4,5-di-(4-fluorophenyl)-2-isopropyl-imidazol-1-yl]-prop-1-en-1-yl]-4-hydroxy-tetrahydropyran-2-one
8-[4,5-di-(4-fluorophenyl)-2-isopropyl-imidazol-1-yl]-3,5-dihydroxy-6-octenoic acid
6-[2-[5-(4-fluorophenyl)-4-(4-methylphenyl)-2-isopropyl-imidazol-1-yl]-ethyl]-4-hydroxy-tetrahydropyran-2-one
7-[5-(4-fluorophenyl)-4-(4-methylphenyl)-2-isopropyl-imidazol-1-yl]-3,5-dihydroxy-heptanoic acid
6-[3-[5-(4-fluorophenyl)-4-(4-methylphenyl)-2-isopropyl-imidazol-1-yl]-prop-1-en-1-yl]-4-hydroxy-tetrahydropyran-2-one
8-[5-(4-fluorophenyl)-4-(4-methylphenyl)-2-isopropyl-imidazol-1-yl]-3,5-dihydroxy-6-octenoic acid
6-[2-(4,5-dimethyl-2-phenyl-imidazol-1-yl)-ethyl]-4-hydroxy-tetrahydropyran-2-one
7-(4,5-dimethyl-2-phenyl-imidazol-1-yl)-3,5-dihydroxy-heptanoic acid
6-[3-(4,5-dimethyl-2-phenyl-imidazol-1-yl)-prop-1-en-1-yl]-4-hydroxy-tetrahydropyran-2-one
8-(4,5-dimethyl-2-phenyl-imidazol-1-yl)-3,5-dihydroxy-6-octenoic acid
6-[2-[4-(4-chlorophenyl)-5-(4-fluorophenyl)-2-isopropyl-imidazol-1-yl]-ethyl]-4-hydroxy-tetrahydropyran-2-one
7-[4-(4-chlorophenyl)-5-(4-fluorophenyl)-2-isopropyl-imidazol-1-yl]-3,5-dihydroxy-heptanoic acid
6-[3-[4-(4-chlorophenyl)-5-(4-fluorophenyl)-2-isopropyl-imidazol-1-yl]-prop-1-en-1-yl]-4-hydroxy-tetrahydropyran-2-one
8-[4-(4-chlorophenyl)-5-(4-fluorophenyl)-2-isopropyl-imidazol-1-yl]-3,5-dihydroxy-6-octenoic acid
6-[2-(4,5-diphenyl-2-methyl-imidazol-1-yl)-ethyl]-4-hydroxy-tetrahydropyran-2-one
7-(4,5-diphenyl-2-methyl-imidazol-1-yl)-3,5-dihydroxy-heptanoic acid
6-[3-(4,5-diphenyl-2-methyl-imidazol-1-yl)-propen-1-en-1-yl]-4-hydroxy-tetrahydropyran-2-one
8-(4,5-diphenyl-2-methyl-imidazol-1-yl)-3,5-dihydroxy-6-octenoic acid
6-[2-[5-(4-chlorophenyl)-4-(4-fluorophenyl)-2-isopropyl-imidazol-1-yl]-ethyl]-4-hydroxy-tetrahydropyran-2-one
7-[5-(4-chlorophenyl)-4-(4-fluorophenyl)-2-isopropyl-imidazol-1-yl]-3,5-dihydroxy-heptanoic acid
6-[3-[5-(4-chlorophenyl)-4-(4-fluorophenyl)-2-isopropyl-imidazol-1-yl]-prop-1-en-1-yl]-4-hydroxy-tetrahydropyran-2-one
8-[5-(4-chlorophenyl)-4-(4-fluorophenyl)-2-isopropyl-imidazol-1-yl]-3,5-dihydroxy-6-octenoic acid
6-[2-[4,5-di-(4-fluorophenyl)-2-cyclobutyl-imidazol-1-yl]-ethyl]-4-hydroxy-tetrahydropyran-2-one
7-[4,5-di-(4-fluorophenyl)-2-cyclobutyl-imidazol-1-yl)-3,5-dihydroxy-heptanoic acid
6-[3-[4,5-di-(4-fluorophenyl)-2-cyclobutyl-imidazol-1-yl]-prop-1-en-1-yl]-4-hydroxy-tetrahydropyran-2-one
8-[4,5-di-(4-fluorophenyl)-2-cyclobutyl-imidazol-1-yl]-3,5-dihydroxy-6-octenoic acid
6-[2-[4,5-di-(4-fluorophenyl)-2-tert-butyl-imidazol-1-yl]-ethyl]-4-hydroxy-tetrahydropyran-2-one
7-[4,5-di-(4-fluorophenyl)-2-tert-butyl-imidazol-1-yl)-3,5-dihydroxy-heptanoic acid
6-[3-[4,5-di-(4-fluorophenyl)-2-tert-butyl-imidazol-1-yl]-prop-1-en-1-yl]-4-hydroxy-tetrahydropyran-2-one
8-[4,5-di-(4-fluorophenyl)-2-tert-butyl-imidazol-1-yl]-3,5-dihydroxy-6-octenoic acid
6-[2-[4,5-di-(4-fluorophenyl)-2-cyclopropyl-imidazol-1-yl]-ethyl]-4-hydroxy-tetrahydropyran-2-one
7-[4,5-di-(4-fluorophenyl)-2-cyclopropyl-imidazol-1-yl)-3,5-dihydroxy-heptanoic acid
6-[3-[4,5-di-(4-fluorophenyl)-2-cyclopropyl-imidazol-1-yl]-prop-1-en-1-yl]-4-hydroxy-tetrahydropyran-2-one
8-[4,5-di-(4-fluorophenyl)-2-cyclopropyl-imidazol-1-yl]-3,5-dihydroxy-6-octenoic acid
6-[2-[5-(4-fluorophenyl)-2-isopropyl-4-phenyl-imidazol-1-yl]-ethyl]-4-hydroxy-tetrahydropyran-2-one
7-[5-(4-fluorophenyl)-2-isopropyl-4-phenyl-imidazol-1-yl)-3,5-dihydroxy-heptanoic acid
6-[3-[5-(4-fluorophenyl)-2-isopropyl-4-phenyl-imidazol-1-yl]-prop-1-en-1-yl]-4-hydroxy-tetrahydropyran-2-one
8-[5-(4-fluorophenyl)-2-isopropyl-4-phenyl-imidazol-1-yl]-3,5-dihydroxy-6-octenoic acid
6-[2-[4,5-di-(4-fluorophenyl)-2-isopropenyl-imidazol-1-yl]-ethyl]-4-hydroxy-tetrahydropyran-2-one
7-[4,5-di-(4-fluorophenyl)-2-isopropenyl-imidazol-1-yl)-3,5-dihydroxy-heptanoic acid
6-[3-[4,5-di-(4-fluorophenyl)-2-isopropenyl-imidazol-1-yl]-prop-1-en-1-yl]-4-hydroxy-tetrahydropyran-2-one
8-[4,5-di-(4-fluorophenyl)-2-isopropenyl-imidazol-1-yl]-3,5-dihydroxy-6-octenoic acid
and the corresponding pharmaceutically acceptable salts, especially sodium salts.

The compounds of formula I inhibit the activity of HMG-CoA reductase and, therefore, they are useful as drugs in the antihypercholesterolemic therapy, in the treatment of atherosclerosis and of hyperlipidaemia.

The compounds of formula I exist in two forms, an open one when R = H (I-A) and a close one (I-B) which are easy interconvertible:

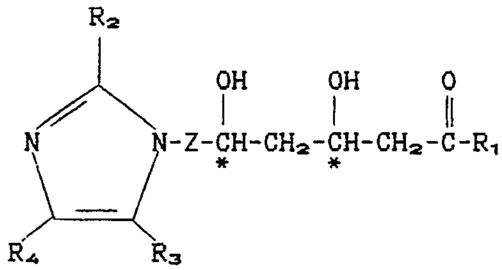

(I-A)

($R_1$=OH, $OR_5$)

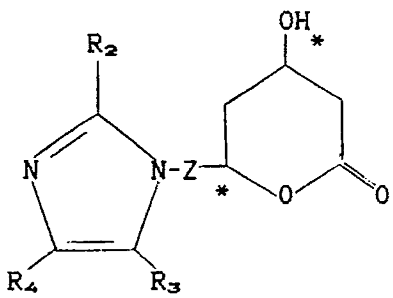

(I-B)

This conversion may be a chemical as well as a biological conversion. Chemically, a compound of formula I-B can be transformed into a salt of the corresponding compound of formula I-A ($R_1$ = OH), for example by basic hydrolysis in mild conditions; the transformation of a compound of formula I-A into the corresponding compound of formula I-B can be carried out, instead, under acidic catalysis or by heating.

Furthermore, a biological conversion of the mevalolactone ring (compound I-B) into the corresponding acid (compound I-A, $R_1$ = OH) usually occurs.

In this connection, it is worth noting that both compounds I-A and compounds I-B are pharmaceutically active.

The preparation of the compounds of formula I is carried out by reacting an imidazole of formula

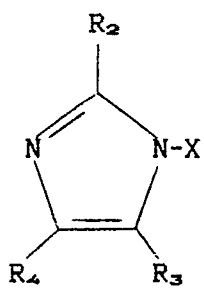

(II)

wherein

$R_2$, $R_3$ and $R_4$ have the above reported meanings;

X is a hydrogen atom or an alkali metal (preferably lithium or sodium) or a tetraalkylammonium group,

with a compound of formula

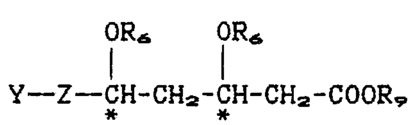

(III)

wherein

Z has the above reported meanings;

$R_6$ is a hydrogen atom or a protecting group of the hydroxy selected among trisubstituted silyl groups or the two $R_6$ together are a

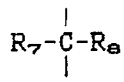

group wherein $R_7$ and $R_8$, the same or different, are hydrogen atoms, alkyl groups or phenyl groups;

$R_9$ is a $C_1$-$C_4$ alkyl or a benzyl;

Y is a leaving group or a group convertible into a leaving group selected among chlorine, bromine, iodine, hydroxy, triphenylmethoxy, methylsulfonyloxy, trifluoromethylsulfonyloxy, benzenesulfonyloxy and p.toluenesulfonyloxy;

the carbon atoms marked by an asterisk are in R or S configuration and syn relative configuration.

The compounds of formula II wherein X=H are known in the literature or they can be easily prepared by known methods.

As an example, Japanese patent application No. 60/56961 [C.A., 103:87878j], J. Am. Chem. Soc., 95, 2695, (1973), J. Chem. Soc., 78, 307, (1956) and J. Heterocycl. Chem., 10, 697, (1973) can be cited. The compounds of formula II wherein X=Na or Li are prepared in the reaction environment by reacting the imidazole compound (II, X=H) with the corresponding alkali hydride under nitrogen atmosphere.

The compounds of formula III wherein Z=$-(CH_2)_2-$ (m=2) have been described in the European patent application No. 352864 (Zambon Group S.p.A.), while the compounds of formula III wherein Z=$-CH_2-$ (m=1), $-(CH)_2)_3-$ (m=3) or $-CH_2-CH=CH-$ are new and they are a further object of the present invention. Their preparation is described hereinafter (see scheme A).

The reaction between compound II and compound III is carried out in an inert solvent selected, in particular, among aprotic dipolar solvents optionally in admixture with a chlorinated solvent, at a temperature between -20°C and 50°C and under anhydrous conditions. Preferably, $R_6$ is different from hydrogen in compound III.

By condensation between compound II and compound III, the compounds of formula I-A as esters ($R_1=OR_5$) and with the hydroxyl groups protected as $OR_6$ groups are obtained.

The deprotection of the hydroxyl groups according to known methods (for example in acetic acid) and, when desired, the hydrolysis of the ester group give the compounds I-A in which $R_1=OR_5$ and $R_1=OH$ respectively. The latter compounds, if desired, can be salified according to known methods, by treatment with the suitable base.

On the contrary, by treating the compound obtained by the condensation between compound II and compound III with aqueous hydrofluoric acid in the presence of water-soluble organic solvents (for example acetonitrile) at a temperature between 0°C and 70°C the compounds of formula I-B are obtained. The salts of the compounds of formula I may be also prepared by treating a compound of formula I-B with a base.

The preparation of the compounds of formula III is carried out according to the reactions reported in the following scheme A and commented hereinafter.

Scheme A

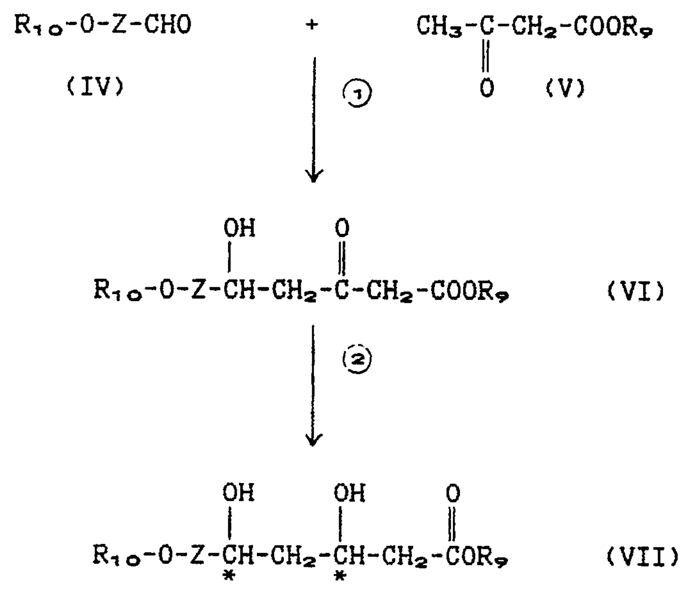

(IV) (V) (VI) (VII)

wherein

$R_9$ and Z have the above reported meanings;

$R_{10}$ is a hydroxy protecting group selected, for example, between tetrahydropyranyl and triphenylmethyl;

the carbon atoms marked by an asterisk are in R or S configuration and in syn relative configuration.

Reaction 1 in scheme A consists in reacting the known aldehydes of formula IV with an acetoacetic ester (V).

The reaction is carried out by preparing in situ the dianion of the acetoacetic ester by treatment with strong bases (lithium diisopropylamide or sodium hydride followed by butyl lithium) and then by adding a solution of the protected aldehyde (IV) in an ethereal solvent (tetrahydrofuran, dimethoxyethane) at a temperature between -15° C and 30° C in an anhydrous environment and under inert gas atmosphere. Similar reactions have been described in a paper published on Can. J. Chem., 52, 2157, (1974).

Thus, compound VI is obtained and it is stereoselectively reduced (reaction 2 in scheme A) giving compound VII.

The stereospecific reduction is carried out by using a borohydride (preferably sodium or zinc borohydride) optionally in the presence of a trialkylborane (for example triethylborane) or of an alkoxydialkylborane (for example methoxydiethylborane) in a solvent inert under the reaction conditions, for example an alcohol (methanol, ethanol) or an ether (dimethoxyethane or tetrahydrofuran) or mixtures thereof, under inert atmosphere and at a temperature between -80° C and 30° C.

The compounds of formula III are prepared from compound VII. For example, the hydroxyl groups can be protected by silylation with the corresponding silylchloride, preferably a hindered silylchloride such as diphenyl-tert.butylsilylchloride in the presence of a base and in an inert solvent.

The transformations of the $R_{10}$-O- group into other groups comprised among the meanings of Y are carried out by known methods.

For example, when $R_{10}$ is tetrahydropyranyl, by treatment with acids in an inert solvent, the hydroxyl group is deprotected and then the compounds of formula III wherein Y = OH are obtained.

The reaction of these latters with a sulfonic acid halide in an inert solvent and in the presence of a base yields the compounds of formula III wherein Y is a methylsulfonyloxy, a trifluoromethylsulfonyloxy, a benzenesulfonyloxy or a p.toluenesulfonyloxy group.

The preparation of the compounds of formula III wherein Y is a chlorine, bromine or iodine atom can be carried out from the corresponding alcohol by reaction with $Cl_2$, $Br_2$ or $I_2$ in the presence of triphenylphosphine in an inert solvent and at a temperature between 0° C and 60° C.

The compounds of formula III wherein the $R_6$ together are a

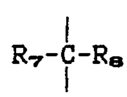

group are prepared by ketalization that is by reacting a compound of formula III (wherein $R_6 = H$) or of formula VII with a carbonyl compound of formula $R_7$-C(O)-$R_8$ wherein $R_7$ and $R_8$ have the above reported meanings.

Alternatively, the preparation of the compounds of formula I is carried out by reacting an imidazole of formula II with a compound of formula VIII

$$Y—Z—A \qquad \text{(VIII)}$$

wherein

- Y and Z have the above reported meanings;
- A is a masked aldehyde group, for example a CN group, an acetal group or a group easily convertible into an aldehyde group such as, for example, a protected alcohol, a carboxyl, an ester or an amido group.

Obviously, the selected group A will be compatible with the substituents of the imidazole.

The reaction between a compound of formula II and a compound of formula VIII is carried out under the same reaction conditions used for the condensation between compound II and compound III.

By the above reaction the compounds of formula

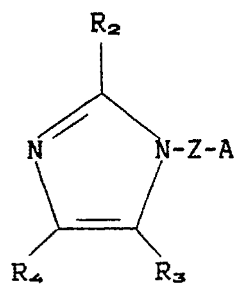

(IX)

wherein Z, A, $R_2$, $R_3$ and $R_4$ have the above reported meanings, are obtained.

The compounds of formula IX can be easily converted into the corresponding compounds of formula

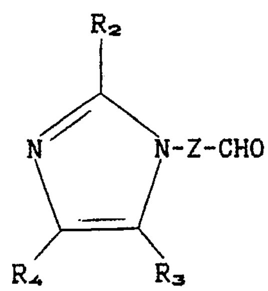

(X)

wherein Z, $R_2$, $R_3$ and $R_4$ have the above reported meanings;
by using known reactions depending on the nature of A.

The compounds of formula X can be converted into the compounds of formula I-A wherein $R_1 = OR_5$ by the series of reactions reported in scheme B.

Scheme B

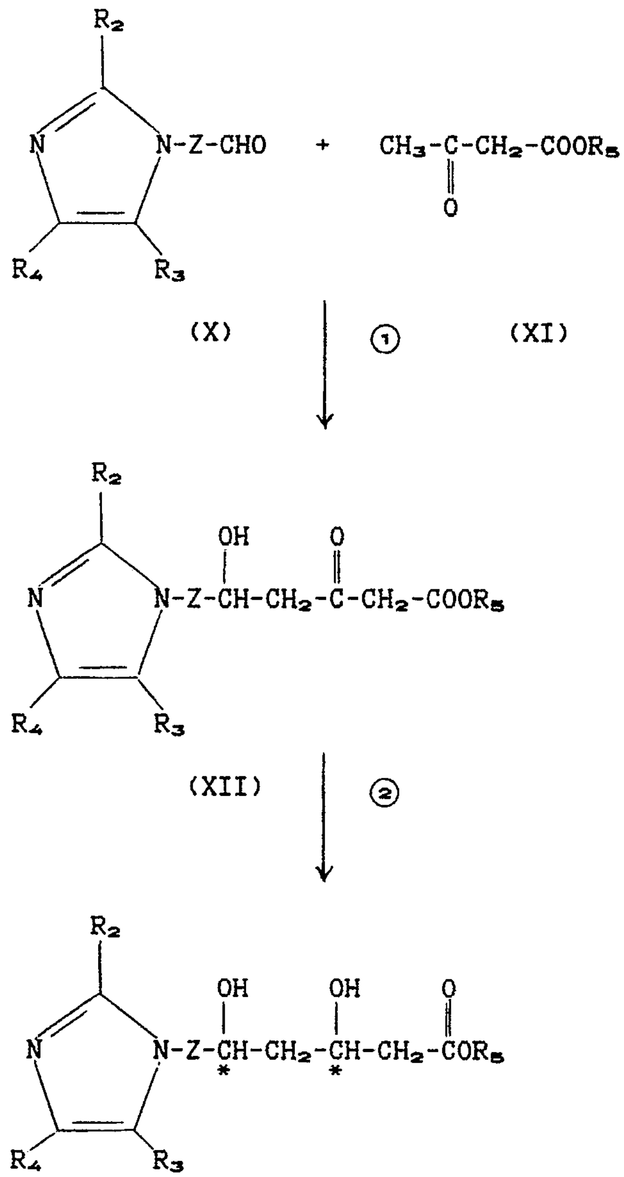


I-A ($R_1 = OR_5$)

wherein $R_2$, $R_3$, $R_4$, $R_5$ and Z have the above reported meanings;
the carbon atoms marked by an asterisk are in R or S configuration and in syn relative configuration.

Reactions 1 and 2 in scheme B are carried out analogously to reactions 1 and 2 in scheme A, which have been already described and commented.

Then, the compounds of formula I-A wherein $R_1 = OR_5$ can be transformed into the compounds of formula I-A wherein $R_1 = OH$, into the compounds of formula I-B or into the salts thereof according to known reactions.

An alternative process for the preparation of the compounds of formula I comprises known cyclization reactions of amines to give imidazoles as reported, for example, in "Rodd's Chemistry of Carbon Compounds", II Ed., IVc, 124, (1986), Elsevier, starting from compounds of formula

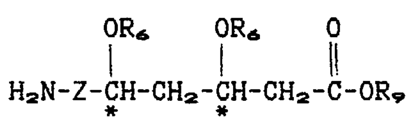

(XIII)

wherein Z, $R_6$ and $R_9$ have the above reported meanings;
the carbon atoms marked by an asterisk are in R or S configuration and in syn relative configuration.

The compounds of formula XIII are new and they are a further object of the present invention.

Their preparation is reported in scheme C.

Scheme C

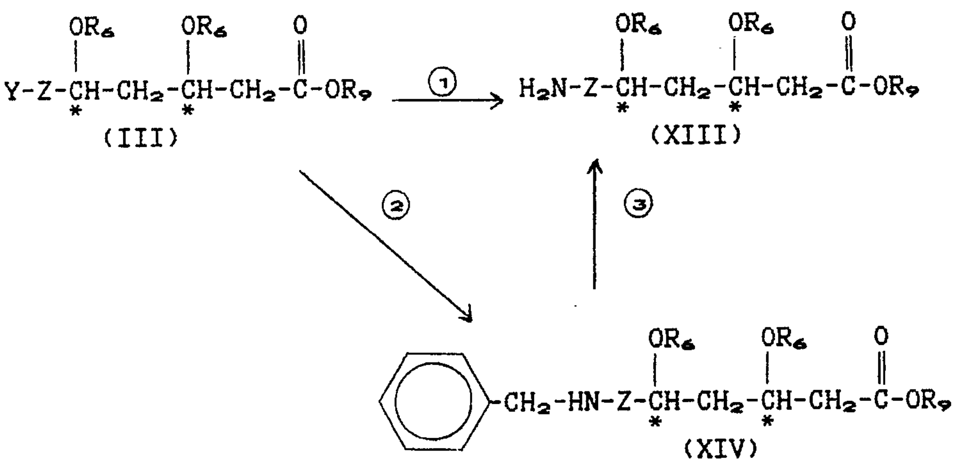

wherein Y, Z, $R_6$ and $R_9$ have the above reported meanings;
the carbon atoms marked by an asterisk are in R or S configuration and in syn relative configuration.

Reactions 1 and 2 in scheme C are carried out by reacting the compounds of formula III with ammonia or with benzylamine respectively in an inert solvent selected, in particular, among aprotic dipolar solvents optionally in admixture with a chlorinated solvent, at a temperature between -20° C and 50° C and under anhydrous conditions. The route through reactions 2 and 3 is used when $Z=(CH_2)_m$.

The debenzylation of the benzyl derivative of formula XIV is carried out by known methods, for example with formic acid in ethanol in the presence of palladium on charcoal at 50° C in order to give the compound of formula XIII.

The compounds of formula I are inhibitors of the enzyme HMG-CoA reductase and, consequently, they can be used in the pharmaceutical field as antihypercholesterolemic and antiatherosclerotic agents. The pharmacological activity of the compounds object of the present invention was evaluated by in vitro tests (example 14).

For the practical use in therapy the compounds of formula I can be used in the form of pharmaceutical compositions which are another object of the present invention. The suitable pharmaceutical compositions depend on the selected administration route.

Examples of such compositions are solid pharmaceutical compositions for oral use such as capsules, tablets and granulates, liquid pharmaceutical compositions for parenteral use such as solutions or freeze-dried powders to be diluted at the moment of use.

The compositions can contain one or more compounds of formula I as active ingredient together with suitable excipients for pharmaceutical use depending on the kind of the composition.

The preparation of the compositions is carried out according to traditional techniques.

Optionally, the compositions object of the present invention can oontain an association of a compound of formula I with another active ingredient. For this purpose, bile acid sequesterings, nicotinic acid derivatives and cholesterolacyltransferase (ACAT) inhibitors are particularly suitable.

The doses of the compounds of formula I to be administered change depending on several factors such as the selected composition, the symptoms and the age of the patient.

In any case, the daily dose will be between 10 and 500 mg to be administered in a single dose or in repeated doses.

In order to better illustrate the present invention, the following examples are given.

Example 1

Preparation of 4,5-di-(4-fluorophenyl)-2-isopropyl-imidazole

Ammonium acetate (1.6 g; 20.31 mmol) was added to a solution of di-(4-fluorophenyl)-ethanedione

(0.365 g; 1.483 mmol) and of isobutyraldehyde (150 µl; 1.631 mmol) in acetic acid (7.5 ml). The mixture was heated under reflux for 1.5 hours.

At the end, the reaction mixture was diluted with water (20 ml), basified with NaOH at 40%, and the mixture was extracted with ethyl acetate (3 x 20 ml).

The combined organic phases were washed with a saturated sodium chloride solution (40 ml) and dried on anhydrous sodium sulfate. The solvent was evaporated obtaining a crude compound (0.5 g) which was collected with warm ethyl ether.

After cooling the solid was filtered obtaining the title compound as a white solid (m.p. 253-255 °C).
$^1$H-NMR (60 MHz, DMSO): δ (ppm): 1.33 (6H, d); 3.07 (1H, m); 6.90-7.80 (8H, m).
Mass (chemical ionization, positive ions, isobutane): m/e 299 $(M+1)^+$
By working in a similar way the following compounds were prepared:
4,5-di-(4-fluorophenyl)-2-cyclobutyl-imidazole
$^1$H-NMR (300 MHz, $CDCl_3$): δ (ppm): 1.85-2.10 (2H, m); 2.38 (4H, m); 3.64 (1H, m); 6.99 (4H, t); 7.40 (4H, dd).
Mass (chemical ionization, positive ions, ammonia): m/e 311 $(M+1)^+$
4,5-di-(4-fluorophenyl)-2-cyclopropyl-imidazole
m.p. 250-252 °C
$^1$H-NMR (300 MHz, DMSO): δ (ppm): 0.93 (4H, m); 1.96 (1H, m); 7.08 (2H, t); 7.23 (2H, t); 7.40 (4H, dd).
Mass (chemical ionization, positive ions, ammonia): m/e 297 $(M+1)^+$
4,5-di-(4-fluorophenyl)-2-isopropenyl-imidazole
$^1$H-NMR (300 MHz, $CDCl_3$): δ (ppm): 2.25 (3H, s); 5.23 (1H, s); 5.61 (1H, s); 7.02 (4H, t); 7.40 (4H, dd).
Mass (chemical ionization, positive ions, ammonia): m/e 297 $(M+1)^+$

Example 2

Preparation of (syn)-3,5-di-(diphenyl-tert.butyl-silyloxy)-7-[4,5-di-(4-fluorophenyl)-2-isopropyl-imidazol-1-yl]-heptanoic acid ethyl ester

4,5-Di-(4-fluorophenyl)-2-isopropyl-imidazole (267 mg; 0.896 mmol), prepared as described in example 1, was added to a suspension of NaH (39 mg; 0.896 mmol) in a mixture of dimethoxyethane:dimethylformamide (ratio 2:1; 4.5 ml) at 0 °C under nitrogen atmosphere.

The suspension was kept under stirring at 50 °C for 30 minutes. Then, the reaction mixture was cooled to room temperature and a 0.2M solution of (syn)-3,5-di-(diphenyl-tert.butyl-silyloxy)-7-trifluoromethylsulfonyloxy-heptanoic acid ethyl ester in carbon tetrachloride (4.5 ml; 0.896 mmol) was added.

The reaction mixture was kept under stirring at room temperature for 72 hours and, then, it was poured into a saturated sodium bicarbonate solution (20 ml) and extracted with ethyl ether (4 x 25 ml).

The combined organic phases were washed with water, dried on anhydrous sodium sulfate and evaporated to dryness.

The resultant crude compound was purified by chromatography on silica gel (230-400 mesh), eluent petroleum ether:ethyl acetate = 85:15, obtaining the pure title compound as an oil.
$^1$H-NMR (300 MHz, $CDCl_3$): δ (ppm): 0.93 (18H, s); 1.16 (3H, t); 1.23 (3H, d); 1.25 (3H, d); 1.40 (2H, m); 1.60 (2H, m); 2.06 (1H, dd); 2.14 (1H, dd); 2.68 (1H, m); 3.49 (2H, m); 3.79 (1H, m); 3.96 (2H, m); 4.06 (1H, m); 6.84 (2H, t); 6.92 (2H, t); 7.04 (2H, dd); 7.25-7.60 (22H, m).
By working in a similar way the following compounds were prepared:
(syn)-3,5-di-(diphenyl-tert.butyl-silyloxy)-7-(4,5-dimethyl-2-phenyl-imidazol-1-yl)-heptanoic acid ethyl ester
$^1$H-NMR (300 MHz, $CDCl_3$): δ (ppm): 0.90 (9H, s); 0.93 (9H, s); 1.14 (3H, t); 1.48 (2H, m); 1.69 (2H, m); 1.85 (3H, s); 2.16 (3H, s); 2.18 (2H, m); 3.64 (2H, m); 3.90 (1H, m); 3.96 (2H, m); 4.13 (1H, m); 7.20-7.45 (17H, m); 7.50-7.60 (8H, m).
(syn)-3,5-di-(diphenyl-tert.butyl-silyloxy)-7-(4,5-diphenyl-2-methyl-imidazol-1-yl)-heptanoic acid ethyl ester
$^1$H-NMR (300 MHz, $CDCl_3$): δ (ppm): 0.90 (9H, s); 0.91 (9H, s); 1.39 (2H, m); 1.60 (2H, m); 2.10 (1H, dd); 2.16 (3H, s); 2.18 (1H, dd); 3.46 (2H, m); 3.77 (1H, m); 3.98 (2H, m); 4.09 (1H, m); 7.05-7.60 (30H, m).
(syn)-3,5-di-(diphenyl-tert.butyl-silyloxy)-7-[4,5-di-(4-fluorophenyl)-2-cyclobutyl-imidazol-1-yl)-heptanoic acid ethyl ester
$^1$H-NMR (300 MHz, $CDCl_3$): δ (ppm): 0.91 (9H, s); 0.94 (9H, s); 1.17 (3H, t); 1.39 (2H, m); 1.62 (2H, m); 1.85-2.60 (8H, m); 3.15-3.30 (1H, m); 3.38 (2H, m); 3.76 (1H, m); 3.98 (2H, m); 4.02 (1H, m); 6.87 (2H, t); 6.92 (2H, t); 7.01 (2H, dd); 7.30-7.60 (22H, m).
(syn)-3,5-di-(diphenyl-tert.butyl-silyloxy)-7-[4,5-di-(4-fluorophenyl)-2-cyclopropyl-imidazol-1-yl)-heptanoic acid ethyl ester

$^1$H-NMR (300 MHz, $CDCl_3$): δ (ppm): 0.90 (9H, s); 0.91 (9H, s); 0.93 (4H, m); 1.40-1.70 (5H, m); 2.10 (1H, dd); 2.20 (1H, dd); 3.60 (2H, m); 3.83 (1H, m); 3.98 (2H, m); 4.03 (1H, m); 6.84 (2H, t); 6.93 (2H, t); 7.03 (2H, dd); 7.25-7.65 (22H, m).

(syn)-3,5-di-(diphenyl-tert.butyl-silyloxy)-7-[4,5-di-(4-fluorophenyl)-2-isopropenyl-imidazol-1-yl)-heptanoic acid ethyl ester

$^1$H-NMR (300 MHz, $CDCl_3$): δ (ppm): 0.86 (9H, s); 0.93 (9H, s); 1.17 (3H, t); 1.40 (1H, m); 1.58 (3H, m); 2.03 (1H, dd); 2.11 (3H, s); 2.14 (1H, dd); 3.60 (2H, m); 3.65 (1H, m); 3.98 (2H, m); 4.12 (1H, m); 5.00 (1H, s); 5.22 (1H, s); 6.86 (2H, t); 6.93 (2H, t); 7.04 (2H, dd); 7.25-7.45 (18H, m); 7.56 (4H, t).

Example 3

Preparation of trans-6-[2-[4,5-di-(4-fluorophenyl)-2-isopropyl-imidazol-1-yl]-ethyl]-4-hydroxy-tetrahydropyran-2-one

A solution of (syn)-3,5-di-(diphenyl-tert.butyl-silyloxy)-7-[4,5-di-(4-fluorophenyl)-2-isopropyl-imidazol-1-yl]-heptanoic acid ethyl ester (0.207 g; 0.2155 mmol), prepared as described in example 2, in a mixture of acetonitrile and of hydrofluoric acid at 40% in water (4.3 ml) was kept under stirring at 50°C for 10 hours.

Hydrofluoric acid was neutralized by powdered sodium bicarbonate, the mixture was diluted with a slight amount of water and with ethyl acetate (20 ml), the salts were filtered and the solution was extracted with ethyl acetate (3 x 20 ml).

The combined organic phases were washed with a saturated sodium chloride solution, dried on sodium sulfate and evaporated.

The resultant crude compound was purified by chromatography on silica gel (230-400 mesh), eluent methylene chloride:acetone = 7:3, yielding the pure compound (0.052 g) as an oil which was collected with warm ethyl ether.

After cooling and filtration, the title compound was obtained as a white solid (m.p. 197-198°C).

$^1$H-NMR (300 MHz, $CDCl_3$): δ (ppm): 1.41 (3H, d); 1.43 (3H, d); 1.50-1.85 (4H, m); 2.55 (1H, dd); 2.63 (1H, dd); 3.11 (1H, m); 4.00 (2H, m); 4.27 (1H, m); 4.51 (1H, m); 6.87 (2H, t); 7.15 (2H, t); 7.30 (2H, dd); 7.35 (2H, dd).

Mass (chemical ionization, positive ions, methane): m/e 441 $(M+1)^+$; 423

By working in a similar way the following compounds were prepared:

trans-6-[2-(4,5-dimethyl-2-phenyl-imidazol-1-yl)-ethyl]-4-hydroxy-tetrahydropyran-2-one

m.p. 132-136°C

$^1$H-NMR (300 MHz, $CDCl_3$): δ (ppm): 1.55 (1H, m); 1.71 (1H, td); 1.70-1.95 (2H, m); 2.17 (3H, s); 2.22 (3H, s); 2.50 (1H, dd); 2.59 (1H, dd); 4.12 (2H, m); 4.22 (1H, m); 4.50 (1H, m); 7.35-7.45 (3H, m); 7.51 (2H, dd).

Mass (chemical ionization, positive ions, isobutane): m/e 315 $(M+1)^+$; 297

trans-6-[2-(4,5-diphenyl-2-methyl-imidazol-1-yl)-ethyl]-4-hydroxy-tetrahydropyran-2-one

m.p. 201-203°C

$^1$H-NMR (300 MHz, $CDCl_3$): δ (ppm): 1.53 (1H, m); 1.60-1.85 (3H, m); 2.52 (3H, s); 2.53 (1H, dd); 2.61 (1H, dd); 3.96 (2H, m); 4.26 (1H, m); 4.50 (1H, m); 7.05-7.20 (3H, m); 7.30-7.50 (7H, m).

Mass (chemical ionization, positive ions, ammonia): m/e 377 $(M+1)^+$; 359

trans-6-[2-[4,5-di-(4-fluorophenyl)-2-cyclobutyl-imidazol-1-yl]-ethyl]-4-hydroxy-tetrahydropyran-2-one

m.p. 130-132°C

$^1$H-NMR (300 MHz, $CDCl_3$): δ (ppm): 1.50-1.85 (4H, m); 1.95-2.20 (2H, m); 2.39 (2H, m); 2.50-2.70 (4H, m); 3.63 (1H, m); 3.86 (1H, m); 3.98 (1H, m); 4.27 (1H, m); 4.50 (1H, m); 6.88 (2H, t); 7.15 (2H, t); 7.28 (2H, dd); 7.37 (2H, dd).

Mass (chemical ionization, positive ions, ammonia): m/e 453 $(M+1)^+$; 435

trans-6-[2-[4,5-di-(4-fluorophenyl)-2-cyclopropyl-imidazol-1-yl]-ethyl]-4-hydroxy-tetrahydropyran-2-one

m.p. 131-132°C

$^1$H-NMR (300 MHz, $CDCl_3$): δ (ppm): 1.04 (2H, m); 1.16 (2H, m); 1.60 (1H, ddd); 1.75 (1H, td); 1.90 (2H, m); 2.55 (1H, dd); 2.64 (1H, dd); 4.11 (2H, m); 4.29 (1H, m); 4.60 (1H, m); 6.86 (2H, t); 7.16 (2H, t); 7.31 (4H, m).

Mass (chemical ionization, positive ions, ammonia): m/e 439 $(M+1)^+$; 421

trans-6-[2-[4,5-di-(4-fluorophenyl)-2-isopropenyl-imidazol-1-yl]-ethyl]-4-hydroxy-tetrahydropyran-2-one

m.p. 170-172°C

$^1$H-NMR (300 MHz, $CDCl_3$): δ (ppm): 1.55 (1H, ddd); 1.71 (1H, td); 1.80 (2H, m); 2.27 (3H, s); 2.54 (1H, dd); 2.63 (1H, dd); 4.05 (1H, m); 4.21 (1H, m); 4.29 (1H, m); 4.46 (1H, m); 5.34 (1H, s); 5.51 (1H, s); 6.88 (2H, t); 7.18 (2H, t); 7.33 (2H, dd); 7.38 (2H, dd).

Mass (chemical ionization, positive ions, ammonia): m/e 439 $(M+1)^+$; 421

Trans-6-[2-[4,5-di-(4-fluorophenyl)-2-isopropyl-imidazol-1-yl]-ethyl]-4-hydroxy-tetrahydropyran-2-one was treated with 0.1M aqueous sodium hydroxide solution.

A portion of the resultant solution was freeze-dried obtaining 7-[4,5-di-(4-fluorophenyl)-2-isopropyl-imidazol-1-yl]-3,5-dihydroxy-heptanoic acid sodium salt.

By acidification of the remaining sodium salt solution with hydrochloric acid up to pH 6 and subsequent extraction, 7-[4,5-di-(4-fluorophenyl)-2-isopropyl-imidazol-1-yl]-3,5-dihydroxy-heptanoic acid was obtained.

Example 4

Preparation of (syn)-3,5-di-(diphenyl-tert.butyl-silyloxy)-7-amino-heptanoic acid ethyl ester

A 0.2M solution of (syn)-3,5-di-(diphenyl-tert.butyl-silyloxy)-7-trifluoromethylsulfonyloxy-heptanoic acid ethyl ester in carbon tetrachloride (15 ml; 3 mmol) was added to a saturated solution of ammonia in chloroform (25 ml) under nitrogen.

The reaction mixture was kept under stirring at room temperature for 22 hours and, then, poured into water (50 ml).

The phases were separated and the organic phase was washed with water (2 x 25 ml), dried on anhydrous sodium sulfate and evaporated. The resultant crude compound was purified by chromatography on silica gel (230-400 mesh), eluent methylene chloride:methanol = 95:5, yielding the pure title compound as an oil.

$^1$H-NMR (300 MHz, $CDCl_3$): δ (ppm): 0.95 (9H, s); 0.97 (9H, s); 1.17 (3H, t); 1.33 (2H, m); 1.74 (2H, m); 2.25 (1H, dd); 2.35 (1H, dd); 2.42 (2H, t); 3.88 (1H, m); 4.00 (2H, m); 4.26 (1H, m); 7.29-7.45 (12H, m); 7.55-7.65 (8H, m).

Example 5

Preparation of (syn)-3,5-di-(diphenyl-tert.butyl-silyloxy)-7-benzylamino-heptanoic acid ethyl ester

A 0.2M solution of (syn)-3,5-di-(diphenyl-tert.butyl-silyloxy)-7-trifluoromethylsulfonyloxy-heptanoic acid ethyl ester in carbon tetrachloride (17 ml; 3.45 mmol) was added to a solution of benzylamine in dimethoxyethane (380 µl; 3.45 mmol), at room temperature and under nitrogen.

The reaction mixture was kept under stirring at room temperature for 36 hours, then, it was poured into a saturated sodium bicarbonate solution (30 ml) and extracted with ethyl ether (4 x 30 ml).

The combined organic phases were washed with water, dried on anhydrous sodium sulfate and evaporated.

The resultant crude compound was purified by chromatography on silica gel (230-400 mesh), eluent petroleum ether:ethyl acetate = 8:2, yielding the pure title compound as an oil.

$^1$H-NMR (300 MHz, $CDCl_3$): δ (ppm): 0.93 (9H, s); 0.95 (9H, s); 1.17 (3H, t); 1.36 (2H, m); 1.74 (2H, m); 2.30 (4H, m); 3.47 (2H, s); 3.87 (1H, m); 3.99 (2H, m); 4.31 (1H, m); 7.15 (2H, d); 7.20-7.45 (15H, m); 7.55-7.65 (8H, m).

Example 6

Preparation of (syn)-3,5-di-(diphenyl-tert.butyl-silyloxy)-7-amino-heptanoic acid ethyl ester

Formic acid at 96% (6 ml) and palladium on charcoal at 10%, (800 mg) were added to a solution of (syn)-3,5-di-(diphenyl-tert.butyl-silyloxy)-7-benzylamino-heptanoic acid ethyl ester (3.96 g; 5.13 mmol), prepared as described in example 5, in ethanol (50 ml).

The suspension was heated at 50°C for 24 hours.

Then, the reaction mixture was evaporated to dryness, the residue was collected with a saturated sodium bicarbonate solution and extracted with ethyl ether.

The combined organic phases were washed with water, dried on anhydrous sodium sulfate and evaporated.

The resultant crude compound was purified by chromatography on silica gel (230-400 mesh), eluent methylene chloride:methanol = 95:5, yielding the pure title compound as an oil.

The physico-chemical characteristics of the compound are the same reported in example 4.

Example 7

Preparation of trans-5-hydroxy-8-tetrahydropyranyloxy-3-oxo-6-octenoic acid ethyl ester

Ethyl acetoacetate (1.5 ml; 11.9 mmol) was added to a suspension of sodium hydride at 55% in mineral oil (570 mg; 13.1 mmol) in tetrahydrofuran (29 ml) at 0° C and under nitrogen atmosphere, while keeping the temperature at about 0° C. After 10 minutes a 1.6M solution of butyllithium in hexane (7.8 ml; 12.5 mmol) was slowly added.

After further 10 minutes at 0° C, a solution of trans-4-tetrahydropyranyloxy-2-butenal [E.J.Corey, J.W.Suggs, Tetrahedron Letters, 2647, (1975)] (2.23 g; 13.1 mmol) in tetrahydrofuran (2 ml) was added fast.

After keeping under stirring for further 10 minutes at the same temperature, the reaction mixture was diluted with a saturated ammonium chloride solution (30 ml) and pH was brought up to about 5-6 with hydrochloric acid.

After extraction with ethyl ether (4 x 50 ml), the combined organic phases were washed with a saturated sodium chloride solution (2 x 50 ml), dried on sodium sulfate and evaporated under vacuum yielding a crude oil (3.5 g) which was purified by chromatography on silica gel (230-400 mesh), eluent methylene chloride:acetone = 9:1.

The pure title compound was obtained as an oil.

$^1$H-NMR (300 MHz, $CDCl_3$): δ (ppm): 1.28 (3H, t); 1.47-1.90 (6H, m); 2.78 (2H, d); 3.47 (2H, s); 3.51 (1H, m); 3.85 (1H, m); 3.97 (1H, dd); 4.14-4.27 (4H, m); 4.63 (1H, m); 5.75 (1H, dd); 5.87 (1H, td).

Example 8

Preparation of trans-(syn)-3,5-dihydroxy-8-tetrahydropyranyloxy-6-octenoic acid ethyl ester

A 1M solution of triethylborane in tetrahydrofuran (4.8 ml; 4.8 mmol) was added to a solution of methanol (8.7 ml) in anhydrous tetrahydrofuran (32 ml) at room temperature and under nitrogen atmosphere.

The solution was kept under stirring at this temperature for one hour, then, it was cooled at -78° C and a solution of trans-5-hydroxy-8-tetrahydropyranyloxy-3-oxo-6-octenoic acid ethyl ester (1.3 g; 4.33 mmol), prepared as described in example 7, in tetrahydrofuran (5 ml) was slowly added under nitrogen atmosphere.

The reaction mixture was kept under stirring at this temperature for 30 minutes; then, sodium borohydride (180 mg; 4.76 mmol) was added and the reaction mixture was kept under stirring at -78° C for 3 hours.

The reaction was stopped with a saturated ammonium chloride solution and the temperature was let arise up to 20° C. The pH was corrected to 5-6 with hydrochloric acid and the resultant mixture was extracted with ethyl acetate (4 x 50 ml).

The combined organic phases were dried on anhydrous sodium sulfate and evaporated.

The resultant residue was collected several times with methanol and, after evaporation to dryness, a crude compound (1.5 g) was obtained and purified by chromatography on silica gel (230-400 mesh), eluent petroleum ether:ethyl acetate = 4:6. The pure title compound was obtained as an oil.

$^1$H-NMR (300 MHz, $CDCl_3$): δ (ppm): 1.26 (3H, t); 1.46-1.90 (8H, m); 2.48 (2H, m); 3.50 (1H, m); 3.84 (1H, m); 3.97 (1H, dd); 4.16 (2H, q); 4.22 (1H, dd); 4.26 (1H, m); 4.42 (1H, m); 4.63 (1H, t); 5.74 (1H, dd); 5.83 (1H, td).

Mass (chemical ionization, positive ions, isobutane): m/e 303 $(M+1)^+$; 219; 201; 183.

Example 9

Preparation of trans-(syn)-3,5-di-(diphenyl-tert.butyl-silyloxy)-8-tetrahydropyranyloxy-6-octenoic acid ethyl ester

A solution of trans-(syn)-3,5-dihydroxy-8-tetrahydropyranyloxy-6-octenoic acid ethyl ester (960 mg; 3.17 mmol), prepared as described in example 8, diphenyl-tert.butylsilylchloride (1.9 ml; 7.62 mmol) and imidazole (865 mg; 12.7 mmol) in dimethylformamide (7.5 ml) was kept under stirring at 70° C for 8 hours. The solution was poured into water (40 ml) and extracted with ethyl ether (4 x 25 ml).

The combined ethereal phases were washed with 0.1M hydrochloric acid (30 ml), with a saturated sodium bicarbonate solution (30 ml) and with a saturated sodium chloride solution (30 ml).

The extracts were dried on anhydrous sodium sulfate and the solvent was evaporated giving a crude compound (3.2 g) which was purified by chromatography on silica gel (230-400 mesh), eluent petroleum ether:ethyl acetate = 93:7.

The pure title compound was obtained as an oil.
$^1$H-NMR (300 MHz, $CDCl_3$): $\delta$ (ppm): 0.95 (18H, s); 1.16 (3H, t); 1.41-1.80 (7H, m); 1.91 (1H, m); 2.32 (1H, dd); 2.43 (1H, ddd); 3.39 (1H, m); 3.61 (1H, m); 3.71 (1H, m); 3.82 (1H, m); 3.99 (2H, m); 4.15-4.30 (2H, m); 4.36 (1H, m); 5.02 (1H, td); 5.31 (1H, m); 7.25-7.75 (20H, m).

Example 10

Preparation of trans-(syn)-3,5-di-(diphenyl-tert.butyl-silyloxy)-8-hydroxy-6-octenoic acid ethyl ester

A solution of trans-(syn)-3,5-di-(diphenyl-tert.butyl-silyloxy)-8-tetrahydropyranyloxy-6-octenoic acid ethyl ester (1.50 g; 1.93 mmol), prepared as described in example 9, and of p.toluenesulfonic acid monohydrate (72 mg; 0.38 mmol) in ethanol (10 ml) was kept under stirring at room temperature for 4 hours.

The solvent was evaporated giving a crude compound (2.6 g) which was purified by chromatography on silica gel (230-400 mesh), eluent petroleum ether:ethyl acetate = 8:2.

The pure title compound was obtained as an oil.
$^1$H-NMR (300 MHz, $CDCl_3$): $\delta$ (ppm): 0.95 (9H, m); 0.97 (9H, s); 1.18 (3H, t); 1.67 (1H, td); 1.89 (1H, td); 2.40 (1H, dd); 2.55 (1H, dd); 3.62 (2H, m); 4.00 (2H, m); 4.22 (2H, m); 5.02 (1H, td); 5.13 (1H, dd); 7.25-7.45 (12H, m); 7.50-7.65 (8H, m).

Example 11

Preparation of trans-(syn)-3,5-di-(diphenyl-tert.butyl-silyloxy)-8-chloro-6-octenoic acid ethyl ester

Methylsulfonylchloride (1.72 ml; 22.15 mmol) was slowly added at 0°C to a solution of trans-(syn)-3,5-di-(diphenyl-tert.butyl-silyloxy)-8-hydroxy-6-octenoic acid ethyl ester (3.5 g; 5.03 mmol), prepared as described in example 10, collidine (2.68 g; 22.15 mmol) and lithium chloride (854 mg; 20.14 mmol) in dimethylformamide (10 ml).

The reaction mixture was kept under stirring for 24 hours at a temperature between 0 and 5°C.

Then, the mixture was poured into a saturated sodium bicarbonate solution (50 ml) and extracted with ethyl ether (3 x 50 ml).

The combined organic phases were washed with water, dried on anhydrous sodium sulfate and evaporated.

The resultant crude compound was purified by chromatography on silica gel (230-400 mesh), eluent petroleum ether:ethyl acetate = 39:1, giving the pure title compound as an oil.
$^1$H-NMR (300 MHz, $CDCl_3$): $\delta$ (ppm): 0.94 (9H, s); 0.98 (9H, s); 1.17 (3H, t); 1.68 (1H, m); 1.87 (1H, m); 2.35 (1H, dd); 2.45 (1H, dd); 3.63 (2H, d); 3.99 (2H, m); 4.20 (2H, m); 5.09 (1H, td); 5.25 (1H, dd); 7.25-7.45 (12H, m); 7.50-7.65 (8H, m).

Example 12

Preparation of trans-(syn)-3,5-di-(diphenyl-tert.butyl-silyloxy)-8-[4,5-di-(4-fluorophenyl)-2-isopropyl-imidazol-1-yl]-6-octenoic acid ethyl ester

At 0°C and under nitrogen atmosphere, 4,5-di-(4-fluorophenyl)-2-isopropyl-imidazole (962 mg; 3.223 mmol), prepared as described in example 1, was added to a suspension of sodium hydride (77 mg; 3.223 mmol) in dimethylsulfoxide (16 ml).

The reaction mixture was kept under stirring at 50°C for 30 minutes and, then, it was cooled to room temperature.

A solution of trans-(syn)-3,5-di-(diphenyl-tert.butyl-silyloxy)-8-chloro-6-octenoic acid ethyl ester (2.3 g; 3.223 mmol), prepared as described in example 11, in dimethylsulfoxide was added and the reaction mixture was kept under stirring at room temperature for 15 hours.

Then, the reaction mixture was poured into a saturated sodium bicarbonate solution (70 ml) and extracted with ethyl ether (4 x 50 ml). The combined organic phases were washed with water, dried on anhydrous sodium sulfate and evaporated to dryness.

The resultant crude compound was purified by chromatography on silica gel (230-400 mesh), eluent petroleum ether:ethyl acetate = 9:1, giving the pure title compound as an oil.
$^1$H-NMR (300 MHz, $CDCl_3$): $\delta$ (ppm): 0.91 (9H, s); 0.95 (9H, s); 1.17 (3H, t); 1.27 (3H, d); 1.28 (3H, d); 1.63 (1H, m); 1.86 (1H, m); 2.32 (1H, dd); 2.39 (1H, dd); 2.66 (1H, m); 3.87 (2H, d); 3.99 (2H, q); 4.13 (1H, m);

4.23 (1H, m); 4.91 (2H, d); 6.88 (2H, t); 6.97 (4H, d); 7.20-7.60 (22H, m).

Example 13

Preparation of trans-(trans)-6-[3-[4,5-di-(4-fluorophenyl)-2-isopropyl-imidazol-1-yl]-prop-1-en-1-yl]-4-hydroxy-tetrahydropyran-2-one

A solution of trans-(syn)-3,5-di-(diphenyl-tert.butyl-silyloxy)-8-[4,5-di-(4-fluorophenyl)-2-isopropyl-imidazol-1-yl]-6-octenoic acid ethyl ester (923 mg; 0.946 mmol), prepared as described in example 12, in a mixture 5:2 of acetonitrile and hydrofluoric acid in water at 40% (22 ml) was kept under stirring at 50° C for 7 hours.

Hydrofluoric acid was neutralized with solid sodium bicarbonate.

The reaction mixture was diluted with a little amount of water and with ethyl acetate (40 ml), the salts were filtered off and the mixture was extracted with ethyl acetate (3 x 40 ml).

The combined organic phases were washed with an aqueous saturated sodium chloride solution, dried on anhydrous sodium sulfate and evaporated.

The resultant crude compound was purified by chromatography on silica gel (230-400 mesh), eluent methylene chloride:acetone = 8:2, giving the title compound as a foamy solid.

$^1$H-NMR (300 MHz, $CDCl_3$): δ (ppm): 1.39 (6H, d); 1.64 (1H, ddd); 1.88 (1H, td); 2.56 (1H, dd); 2.63 (1H, dd); 4.25 (1H, m); 4.37 (2H, d); 5.13 (1H, m); 5.20 (1H, dd); 5.79 (1H, td); 6.88 (2H, t); 7.10 (2H, t); 7.24 (2H, dd); 7.37 (2H, dd).

Mass (chemical ionization, positive ions, ammonia): m/e 453 $(M+1)^+$, 435.

Example 14

Evaluation of activity in vitro

1) Preparation of hepatic microsomes

Male Sprague-Dowley rats (100-125 g) were kept in a cage with a controlled light-dark cycle for 7 days, fed with a standard diet "Altromin®" and with water ad libitum.

The animals were sacrified by decapitation and the livers were immediately drawn and used for the preparation of microsomes according to the procedure described by Shapiro and Rodwell (J. Biol. Chem., 246, 3210, 1971).

The resultant microsomial pellet was suspended in phosphate buffer and preserved at -80° C.

2) In vitro evaluation of HMG-CoA reductase activity

The activity of the enzyme HMG-CoA reductase was evaluated according to the method described by Goldstein and Brawn (Proc. Nat. Acad. Sci. USA, 73, 2564, 1976).

The incubation mixture (200 µl) consisted of: phosphate buffer 100 mM (pH 7.4), dithiothreitol 10 mM, EDTA 10 mM, NADP 25 mM, glucose-6-phosphate 20 mM, glucose-6-phosphate dehydrogenase 3 U/ml, [$^{14}$C]-HMG-CoA (New England Nuclear) 100 µM (0.08 µCi), 600 µg of microsomial proteins.

Compounds of formula I or Mevastatin, as reference compound, were added to the above incubation mixture at different concentrations in order to evaluate the $IC_{50}$ values.

As internal standard [$^3$H]-mevalolactone was used.

Unreacted [$^{14}$C]-HMG-CoA was eliminated as described by Alberts et al. (Proc. Nat. Acad. Sci. USA, 77, 3957, 1980) by using columns containing AG 1-x8 resin (Bio-rad).

Then, [$^{14}$C]-mevalolactone was eluted with distilled water (3 x 750 µl) directly into vials containing 10 ml of "Picofluor-40®" (Packard).

The radioactivity of the samples was measured by a beta-counter series 400 (Packard).

The so calculated $IC_{50}$ values showed that the compounds of formula I, object of the present invention, are about 2-3 times more active than Mevastatin in inhibiting HMG-CoA reductase activity in vitro.

**Claims**

**1.** A compound of formula

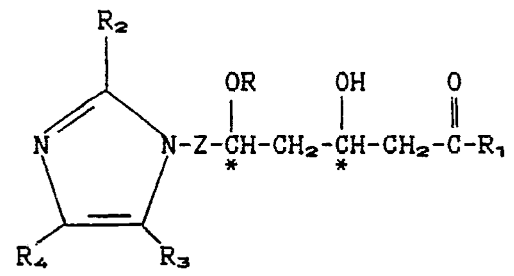

(I)

wherein

| | |
|---|---|
| R | is a hydrogen atom; |
| $R_1$ | is a hydroxyl or an $-OR_5$ group wherein $R_5$ is a $C_1$-$C_4$ alkyl or a benzyl; or R and $R_1$, together, are a single bond between the oxygen atom and the carbonyl group; |
| $R_2$, $R_3$ and $R_4$, | the same or different, are hydrogen atoms, $C_1$-$C_5$ alkyl or alkoxy groups, $C_2$-$C_5$ alkenyl groups, $C_3$-$C_7$ cycloalkyl or cycloalkylmethyl groups, halogen atoms, $CF_3$ or CN groups, phenyl or benzyl optionally substituted by 1 or 2 substituents selected among halogen atoms and $C_1$-$C_4$ alkyl, a pyridyl or N-oxide thereof; |
| Z | is a $-(CH_2)_m-$ group wherein m is 1, 2 or 3 or a $-CH_2-CH=CH-$group; |

the carbon atoms marked by an asterisk are in R or S configuration and in syn relative configuration; and, when $R_1$ is a hydroxyl group, the salts thereof with pharmaceutically acceptable bases.

2. A compound of formula

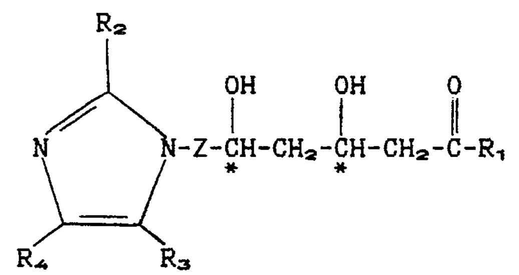

(I-A)

wherein

| | |
|---|---|
| $R_1$ | is a hydroxyl group or a $-OR_5$ group wherein $R_5$ is a $C_1$-$C_4$ alkyl or a benzyl; |
| $R_2$, $R_3$ and $R_4$, | the same or different, are hydrogen atoms, $C_1$-$C_5$ alkyl or alkoxy groups, $C_2$-$C_5$ alkenyl groups, $C_3$-$C_7$ cycloalkyl or cycloalkylmethyl groups, halogen atoms, $CF_3$ or CN groups, phenyl or benzyl optionally substituted by 1 or 2 substituents selected among halogen atoms and $C_1$-$C_4$ alkyl, a pyridyl or N-oxide thereof; |
| Z | is a $-(CH_2)_m-$ group wherein m is 1, 2 or 3 or a $-CH_2-CH=CH-$group; |

the carbon atoms marked by an asterisk are in R or S configuration and in syn relative configuration; and, when $R_1$ is a hydroxyl group, the salts thereof with pharmaceutically acceptable bases.

3. A compound of formula

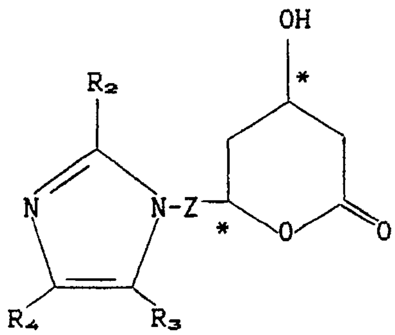

(I-B)

wherein

$R_2$, $R_3$ and $R_4$, the same or different, are hydrogen atoms, $C_1$-$C_5$ alkyl or alkoxy groups, $C_2$-$C_5$ alkenyl groups, $C_3$-$C_7$ cycloalkyl or cycloalkylmethyl groups, halogen atoms, $CF_3$ or CN groups, phenyl or benzyl optionally substituted by 1 or 2 substituents selected among halogen atoms and $C_1$-$C_4$ alkyl, a pyridyl or N-oxide thereof;

Z is a $-(CH_2)_m-$ group wherein m is 1, 2 or 3 or a $-CH_2-CH=CH-$group;

the carbon atoms marked by an asterisk are in R or S configuration and in syn relative configuration.

4. A compound according to claims 1, 2 or 3 in which $R_2$, $R_3$ and $R_4$, the same or different, are hydrogen atoms, $C_1$-$C_4$ alkyl or alkoxy groups, $C_2$-$C_3$ alkenyl groups, $C_3$-$C_5$ cycloalkyl or cycloalkylmethyl groups, phenyl or benzyl optionally substituted by 1 or 2 substituents selected among fluorine, chlorine, bromine atoms and methyl groups.

5. A compound according to claims 1, 2 or 3 in which $R_2$, $R_3$ and $R_4$, the same or different, are methyl, ethyl, propyl, isopropyl, tertbutyl, methoxy, ethoxy, propoxy, isopropoxy, allyl, isopropenyl, cyclopropyl, cyclobutyl, phenyl, 4-fluorophenyl, 4-chlorophenyl, 4-bromophenyl or 4-methylphenyl; Z is a $-(CH_2)_m-$ group wherein m is 2 or a $-CH_2-CH=CH-$ group.

6. A compound of formula

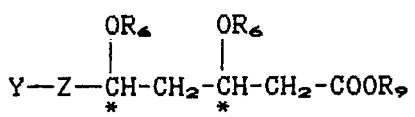

(III)

wherein

Z is a $-(CH_2)_m-$ group wherein m is 1 or 3 or a $-CH_2-CH=CH-$ group;

$R_6$ is a hydrogen atom or a protecting group of hydroxy selected among trisubstituted silyl groups or the two $R_6$ together are a

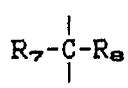

group wherein $R_7$ and $R_8$, the same or different, are hydrogen atoms, alkyl groups or phenyl groups;

$R_9$ is a $C_1$-$C_4$ alkyl or a benzyl;

Y is a leaving group or a group convertible in a leaving group selected among chlorine, bromine, iodine, hydroxy, triphenylmethoxy, methylsulfonyloxy, trifluoromethylsulfonyloxy, benzenesulfonyloxy and p.toluenesulfonyloxy;

the carbon atoms marked by an asterisk are in R or S configuration and syn relative configuration.

7. A compound of formula

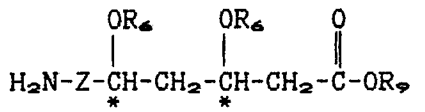

(XIII)

wherein

Z is a $-(CH_2)_m-$ group wherein m is 1, 2 or 3 or a $-CH_2-CH=CH-$group;

$R_6$ is a hydrogen atom or a protecting group of hydroxy selected among trisubstituted silyl groups or the two $R_6$ together are a

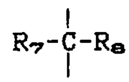

group wherein $R_7$ and $R_8$, the same or different, are hydrogen atoms, alkyl groups or phenyl groups;

$R_9$ is a $C_1$-$C_4$ alkyl or a benzyl.

the carbon atoms marked by an asterisk are in R or S configuration and syn relative configuration.

**8.** A pharmaceutical composition containing a compound according to claim 1, as active ingredient, and a carrier suitable for pharmaceutical use.

**Claims for the following Contracting State: Gr**

**1.** A process for the preparation of compounds of formula

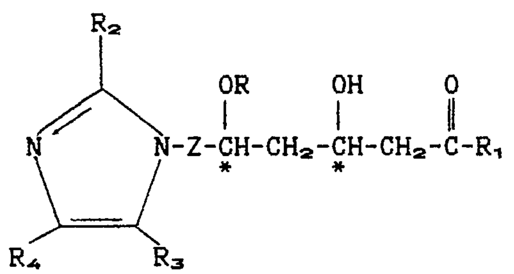

(I)

wherein

R is a hydrogen atom;

$R_1$ is a hydroxyl or an $-OR_5$ group wherein $R_5$ is a $C_1$-$C_4$ alkyl or a benzyl; or R and $R_1$, together, are a single bond between the oxygen atom and the carbonyl group;

$R_2$, $R_3$ and $R_4$, the same or different, are hydrogen atoms, $C_1$-$C_5$ alkyl or alkoxy groups, $C_2$-$C_5$ alkenyl groups, $C_3$-$C_7$ cycloalkyl or cycloalkylmethyl groups, halogen atoms, $CF_3$ or CN groups, phenyl or benzyl optionally substituted by 1 or 2 substituents selected among halogen atoms and $C_1$-$C_4$ alkyl, a pyridyl or N-oxide thereof;

Z is a $-(CH_2)_m-$ group wherein m is 1, 2 or 3 or a $-CH_2-CH=CH-$group;

the carbon atoms marked by an asterisk are in R or S configuration and in syn relative configuration; comprising the condensation between an imidazole of formula

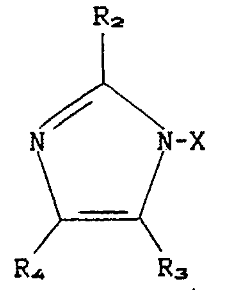

(II)

wherein

$R_2$, $R_3$ and $R_4$ have the above reported meanings;

X is a hydrogen atom or an alkali metal or a tetraalkylammonium group,

and a compound of formula

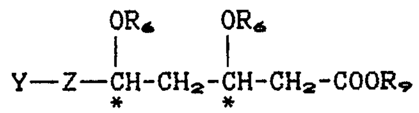

(III)

wherein

Z has the above reported meanings;

$R_6$ is a hydrogen atom or a protecting group of hydroxy selected among trisubstituted silyl groups or the two $R_6$ together are a

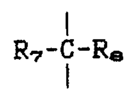

group wherein $R_7$ and $R_8$, the same or different, are hydrogen atoms, alkyl groups or phenyl groups;

$R_9$ is a $C_1$-$C_4$ alkyl or a benzyl;

Y is a leaving group or a group convertible in a leaving group selected among chlorine, bromine, iodine, hydroxy, triphenylmethoxy, methylsulfonyloxy, trifluoromethylsulfonyloxy, benzenesulfonyloxy and p.toluenesulfonyloxy;

the carbon atoms marked by an asterisk are in R or S configuration and syn relative configuration; in an aprotic dipolar solvent optionally in admixture with a chlorinated solvent, at a temperature between -20 °C and 50 °C and under anhydrous conditions.

**2.** A compound of formula

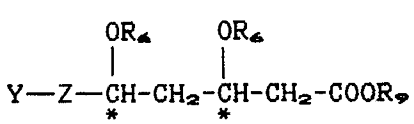

(III)

wherein

Z is a $-(CH_2)_m-$ group wherein m is 1 or 3 or a $-CH_2-CH=CH-$ group;

$R_6$ is a hydrogen atom or a protecting group of hydroxy selected among trisubstituted silyl groups or the two $R_6$ together are a

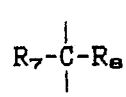

group wherein $R_7$ and $R_8$, the same or different, are hydrogen atoms, alkyl groups or phenyl groups;

$R_9$ is a $C_1$-$C_4$ alkyl or a benzyl;

Y is a leaving group or a group convertible in a leaving group selected among chlorine, bromine, iodine, hydroxy, triphenylmethoxy, methylsulfonyloxy, trifluoromethylsulfonyloxy, benzenesulfonyloxy and p.toluenesulfonyloxy;

the carbon atoms marked by an asterisk are in R or S configuration and syn relative configuration.

3. A compound of formula

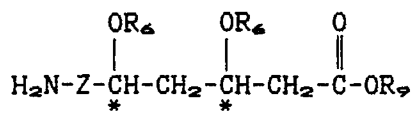

(XIII)

wherein

Z is a $-(CH_2)_m-$ group wherein m is 1, 2 or 3 or a $-CH_2-CH=CH-$group;

$R_6$ is a hydrogen atom or a protecting group of hydroxy selected among trisubstituted silyl groups or the two $R_6$ together are a

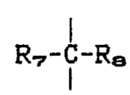

group wherein $R_7$ and $R_8$, the same or different, are hydrogen atoms, alkyl groups or phenyl groups;

$R_9$ is a $C_1$-$C_4$ alkyl or a benzyl.

the carbon atoms marked by an asterisk are in R or S configuration and syn relative configuration.

**Claims for the following Contracting State: Es**

1. A process for the preparation of compounds of formula

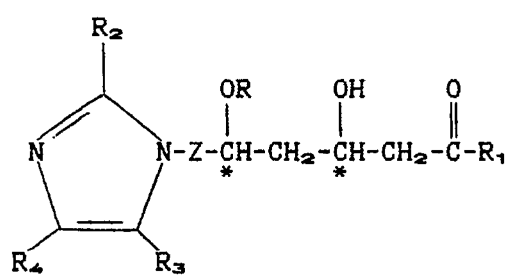

(I)

wherein

R is a hydrogen atom;

$R_1$ is a hydroxyl or an $-OR_5$ group wherein $R_5$ is a $C_1$-$C_4$ alkyl or a benzyl; or R and $R_1$, together, are a single bond between the oxygen atom and the carbonyl group;

$R_2$, $R_3$ and $R_4$, the same or different, are hydrogen atoms, $C_1$-$C_5$ alkyl or alkoxy groups, $C_2$-$C_5$ alkenyl groups, $C_3$-$C_7$ cycloalkyl or cycloalkylmethyl groups, halogen atoms, $CF_3$ or CN groups, phenyl or benzyl optionally substituted by 1 or 2 substituents selected among halogen atoms and $C_1$-$C_4$ alkyl, a pyridyl or N-oxide thereof;

Z is a $-(CH_2)_m-$ group wherein m is 1, 2 or 3 or a $-CH_2-CH=CH-$group;

the carbon atoms marked by an asterisk are in R or S configuration and in syn relative configuration; comprising the condensation between an imidazole of formula

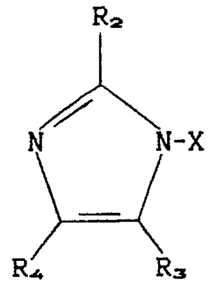

(II)

wherein

$R_2$, $R_3$ and $R_4$ have the above reported meanings;

X is a hydrogen atom or an alkali metal or a tetraalkylammonium group,

and a compound of formula

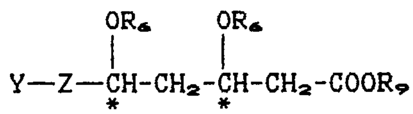

(III)

wherein

Z has the above reported meanings;

$R_6$ is a hydrogen atom or a protecting group of hydroxy selected among trisubstituted silyl groups or the two $R_6$ together are a

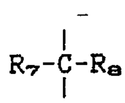

group wherein $R_7$ and $R_8$, the same or different, are hydrogen atoms, alkyl groups or phenyl groups;

$R_9$ is a $C_1$-$C_4$ alkyl or a benzyl;

Y is a leaving group or a group convertible in a leaving group selected among chlorine, bromine, iodine, hydroxy, triphenylmethoxy, methylsulfonyloxy, trifluoromethylsulfonyloxy, benzenesulfonyloxy and p.toluenesulfonyloxy;

the carbon atoms marked by an asterisk are in R or S configuration and syn relative configuration;
in an aprotic dipolar solvent optionally in admixture with a chlorinated solvent, at a temperature between -20° C and 50° C and under anhydrous conditions.

**2.** A process for the preparation of compounds of formula

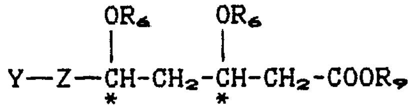

(III)

wherein

Y, $R_6$ and $R_9$ have the meanings reported in claim 1;

Z is a $-(CH_2)_m-$ group wherein m is 1 or 3 or a $-CH_2-CH=CH-$ group;

the carbon atoms marked by an asterisk are in R or S configuration and syn relative configuration;
comprising the stereospecific reduction of a compound of formula

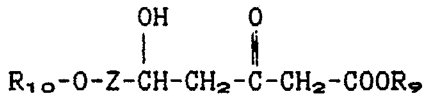

(VI)

wherein

Z and $R_9$ have the above reported meanings;

$R_{10}$ is a hydroxy protective group selected between tetrahydropyranyl and triphenylmethyl;

to give a compound of formula

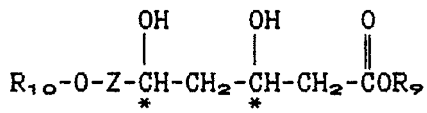

(VII)

wherein

Z, $R_9$ and $R_{10}$ have the above reported meanings;

and its transformation into the corresponding compound of formula III.

**3.** A process for the preparation of compounds of formula

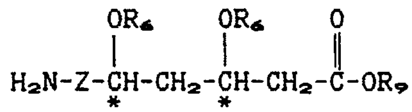

(XIII)

wherein

Z is a $-(CH_2)_m-$ group wherein m is 1, 2 or 3 or a $-CH_2-CH=CH-$group;

$R_6$ is a hydrogen atom or a protecting group of hydroxy selected among trisubstituted silyl groups or the two $R_6$ together are a

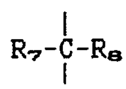

group wherein $R_7$ and $R_8$, the same or different, are hydrogen atoms, alkyl groups or phenyl groups;

$R_9$ is a $C_1$-$C_4$ alkyl or a benzyl;

the carbon atoms marked by an asterisk are in R or S configuration and syn relative configuration; comprising the reaction of a compound of formula

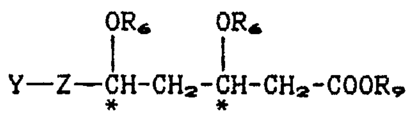

(III)

wherein

Z, $R_6$ and $R_9$ have the above reported meanings;

Y has the meanings reported in claim 1;

with ammonia or with benzylamine in an aprotic polar solvent optionally in admixture with a chlorinated solvent, at a temperature between -20 °C and 50 °C and under anhydrous conditions and the optional debenzylation.

European Patent Office

# EUROPEAN SEARCH REPORT

Application Number

EP 90 12 3759

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 324 347 (HOECHST AG) --- | | C 07 D 233/54<br>A 61 K 31/415<br>C 07 D 233/60<br>C 07 F 7/18 |
| A | EP-A-0 334 014 (BAYER AG) --- | | |
| A | EP-A-0 221 025 (SANDOZ-PATENT-GmbH) --- | | |
| A | EP-A-0 302 253 (HOECHST AG) --- | | |
| A | EP-A-0 244 362 (CIBA-GEIGY AG) --- | | |
| D,A | WO-A-8 607 054 (SANDOZ-ERFINDUNGENVERWALTUNGSGESELLSCHAF-T mbH) --- | | |
| D,A | WO-A-8 402 131 (SANDOZ AG) --- | | |
| D,A | EP-A-0 179 559 (WARNER-LAMBERT CO.) --- | | |
| P,A | EP-A-0 354 418 (HOECHST AG) --- | | |
| P,A | EP-A-0 361 273 (HOECHST AG) --- | | TECHNICAL FIELDS SEARCHED (Int. Cl.5) |
| P,D,A | EP-A-0 352 864 (ZAMBON GROUP S.p.A.) ------ | | C 07 D 233/00<br>A 61 K 31/00<br>C 07 F 7/00 |

The present search report has been drawn up for all claims

| Place of search | Date of completion of search | Examiner |
|---|---|---|
| The Hague | 29 March 91 | DE BUYSER I.A.F. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document